# EUROPÄISCHE PATENTANMELDUNG

(11) **EP 3 058 967 A1**
(43) Veröffentlichungstag der Anmeldung: **24.08.2016**
(21) Anmeldenummer: 15155899.6
(22) Anmeldetag: 20.02.2015
(51) Int. Cl.: A61M 1/06

(54) **Brusthaubeneinheit und Konnektor für eine Brustpumpe**

(71) Anmelder: Medela Holding AG, 6340 Baar (CH)
(72) Erfinder: Schlienger, André, 8933 Maschwanden (CH); Furrer, Etienne, 6332 Hagendorn (CH); Muther, Marcel, 6030 Ebikon (CH)
(74) Vertreter: Clerc, Natalia

(57) **Zusammenfassung**

Eine Brusthaubeneinheit zur Verwendung in einer Vorrichtung zum Abpumpen von menschlicher Muttermilch weist eine Brusthaube (2) zur Anlage an eine Mutterbrust, einen Milchsammelbehälter (3) zur Aufnahme der abgepumpten Muttermilch und einen Konnektor (1) zur Verbindung der Brusthaube (2) mit einer Vakuumpumpe der Vorrichtung und mit dem Milchsammelbehälter (3) auf. Die Brusthaube (2) weist eine erste Längsmittelachse (A₁) und der Milchsammelbehälter (3) eine zweite Längsmittelachse (A₂) auf, wobei die mit dem Konnektor (1) verbundene Brusthaube (2) relativ zum ebenfalls mit dem Konnektor (1) verbundenen Milchsammelbehälter (3) bewegbar ist. Der Konnektor (1) ist aus mindestens einem ersten Konnektorteil (10, 11) und einem zweiten Konnektorteil (12) zusammengesetzt, wobei das erste Konnektorteil (10, 11) den Milchsammelbehälter (3) hält und das zweite Konnektorteil (12) die Brusthaube (2). Das erste Konnektorteil (10, 11) ist um eine Schwenkachse (A₃) relativ zum zweiten Konnektorteil (12) schwenkbar, wobei diese Schwenkachse (A₃) weder die erste noch die zweite Längsmittelachse (A₁, A₂) ist. Der schwenkbare und als Rotationskopf ausgebildete Konnektor ermöglicht ein Abpumpen bei zurückgelehnter Körperhaltung der Mutter in diversen Positionen der Brusthaube, wobei der Milchsammelbehälter in einer annähernd aufrechten Position verbleibt.

## Beschreibung

### TECHNISCHES GEBIET

Die vorliegende Erfindung betrifft eine Brusthaubeneinheit sowie einen Konnektor einer Brustpumpe zum Abpumpen von menschlicher Muttermilch.

### STAND DER TECHNIK

Brustpumpensysteme zum Abpumpen von menschlicher Muttermilch sind wohlbekannt. Sie weisen eine manuell oder elektromotorisch betriebene Vakuumpumpe, mindestens eine Brusthaube zur Auflage auf die Mutterbrust und einen Milchsammelbehälter auf, in welchem die abgepumpte Milch aufgefangen wird. Die Brusthaube ist mit der Vakuumpumpe direkt oder über eine Saugleitung verbunden, so dass in der Brusthaube ein zyklisch variierender Unterdruck an die Brust angelegt werden kann, um die Milch aus der Brust abzupumpen.

Bekannte Brusthauben sind in einem Verbindungsstück, auch Adapter oder Konnektor genannt, lösbar gehalten oder fest mit diesem verbunden. Der Konnektor dient zur Verbindung mit einem Milchsammelbehälter und mit einer Vakuumpumpe. Die Pumpe kann dabei über eine Saugleitung mit dem Konnektor verbunden sein oder direkt an diesem angeordnet sein. Auch der Milchsammelbehälter, üblicherweise ein Beutel oder eine Flasche, ist direkt lösbar am Konnektor befestigt oder über eine Milchleitung mit diesem verbunden.

Ein sehr einfach ausgebildeter Konnektor weist im Wesentlichen einen rohrförmigen Aufnahmeteil zur Aufnahme der Brusthaube, eine Öffnung zur Verbindung mit der Saugleitung und einen einstückig am Aufnahmeteil und in einem Winkel dazu angeformten rohrförmigen Milchanschluss auf. Der Milchanschluss verfügt über ein Innengewinde zur Verbindung mit einem Gewindehals einer Milchflasche. Eine derartige Brusthaube ist in WO 01/47577 gezeigt.

Es ist ferner bekannt, im Konnektor eine Medientrennmembran anzuordnen, welche das von der Vakuumpumpe zyklisch erzeugte Vakuum auf die Brusthaube überträgt und gleichzeitig den Pumpenbereich vor Verunreinigungen durch Milch und Kontamination durch Bakterien schützt. Diese Konnektoren sind üblicherweise komplizierter aufgebaut.

US 8 444 596 zeigt einen derartigen Konnektor mit einem Drehverschlussdeckel, damit die Medientrennmembran auf der oberen Seite des Konnektors eingelegt werden kann.

Die meisten Brustpumpensysteme sind so ausgebildet, dass die Mutter während des Abpumpens zwingend aufrecht sitzen muss, damit die Brusthaube genügend dicht an der Brust anliegt und die Milch von der Brusthaube in den Milchsammelbehälter fliessen kann.

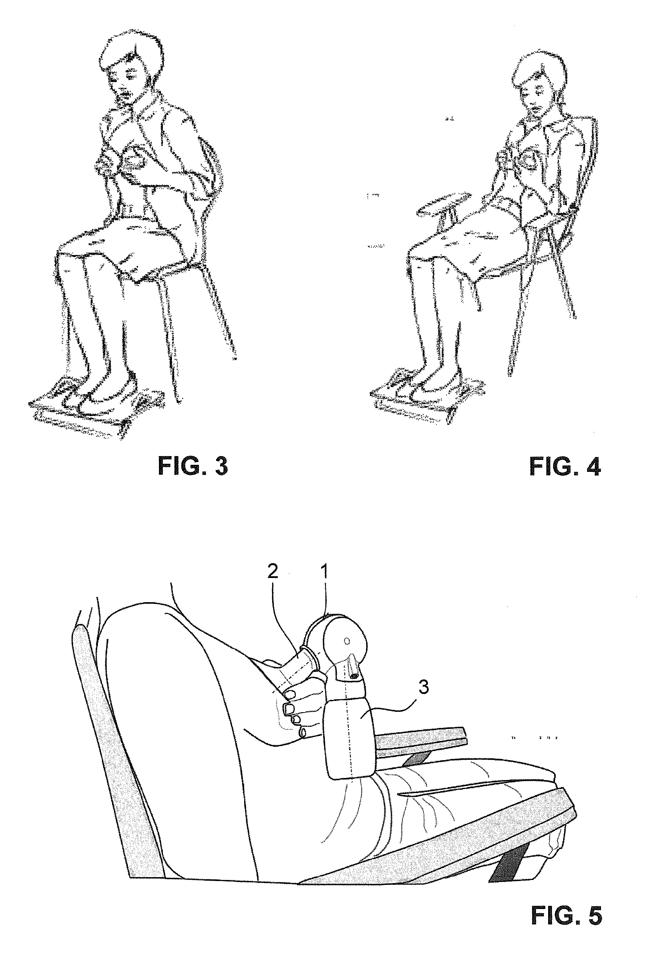

Dies führt oft dazu, dass sich die Mutter während des Abpumpens kaum zu bewegen wagt und steif dasitzt. Dies ist in Figur 3 dargestellt. Diese für sie ungemütliche Körperhaltung kann sich auf das Ergebnis des Abpumpens auswirken.

US 2006/0116632 schlägt deshalb vor, einen Anschlussstutzen der Brusthaube in einem Winkel zum kegelstumpfartigen Trichter anzuordnen. In diesem Fall kann die Mutter jedoch nur noch in zurückgelehnter Haltung abpumpen.

Der Stand der Technik offenbart auch flexiblere Systeme, welche der Mutter in einem geringen Mass eine bequeme Haltung während des Abpumpens erlauben.

US 2009/0171270 offenbart beispielsweise eine Brusthaube, welche einen balgförmigen Anschlussstutzen aufweist. Dadurch lässt sich die Brusthaube in einem geringen Mass in ihrem Winkel verändern.

US 5 571 084 zeigt einen Konnektor mit einem balgförmigen Hals, welcher auf der Milchflasche befestigt ist. Auch hier lässt sich der Winkel zwischen Brusthaube und Milchflasche begrenzt verändern.

EP 1 034 807 offenbart einen Konnektor, in welchem die Brusthaube schwenkbar gehalten ist. WO 2014/068066 offenbart verschiedene Lösungen, bei welchen der Konnektor schwenkbar zum Milchsammelbehälter angeordnet ist.

Es hat sich in der Praxis gezeigt, dass die Mütter, selbst wenn das Brustpumpensystem eine bequemere Haltung beim Abpumpen ermöglicht, diese trotzdem nicht wählen. Grund hierfür ist, dass die Mutter automatisch versucht, die Milchflasche in einer möglichst senkrechten Lage zu halten.

### DARSTELLUNG DER ERFINDUNG

Es ist deshalb eine Aufgabe der Erfindung, eine Vorrichtung zu schaffen, welche die Mutter dazu animiert, eine für sie bequeme Haltung während des Abpumpens der Milch einzunehmen.

Diese Aufgabe lösen eine Brusthaubeneinheit mit den Merkmalen des Patentanspruchs 1 sowie ein Konnektor mit den Merkmalen des Patentanspruchs 15.

Die erfindungsgemässe Brusthaubeneinheit zur Verwendung in einer Vorrichtung zum Abpumpen von menschlicher Muttermilch weist eine Brusthaube zur Anlage an eine Mutterbrust und einen Milchsammelbehälter zur Aufnahme der abgepumpten Muttermilch auf. Ferner weist sie einen Konnektor auf zur Verbindung der Brusthaube mit einer Vakuumpumpe der Vorrichtung und mit dem Milchsammelbehälter. Der Konnektor weist einen Milchkanal auf, welcher von der Brusthaube zum Milchsammelbehälter führt. Die Brusthaube hat eine erste Längsmittelachse und der Milchsammelbehälter hat eine zweite Längsmittelachse. Die mit dem Konnektor verbundene Brusthaube ist relativ zum ebenfalls mit dem Konnektor verbundenen Milchsammelbehälter bewegbar. Der Konnektor ist aus mindestens einem ersten Konnektorteil und einem zweiten Konnektorteil zusammengesetzt, wobei das erste Konnektorteil den Milchsammelbehälter hält und das zweite Konnektorteil die Brusthaube hält. Das erste Konnektorteil ist um eine Schwenkachse relativ zum zweiten Konnektorteil schwenkbar. Diese Schwenkachse ist dabei weder die erste noch die zweite Längsmittelachse.

Dank dieser Anordnung der Schwenkachse lässt sich die Brusthaube um die zweite Längsmittelachse des Milchsammelbehälters schwenken, wobei sie dabei gleichzeig ihren Neigungswinkel in Bezug auf den Milchsammelbehälter ändert. Die Mutter kann dadurch eine für sie bequeme und dichte Position der Brusthaube finden, ohne dass der Milchsammelbehälter allzu stark geneigt werden muss. Die Mutter kann auch in zurückgelehnter und halb oder ganz liegender Stellung Milch abpumpen und trotzdem den Milchsammelbehälter in einer annähernd aufrechten oder vertikalen Position halten. Dies ist in Figur 4 dargestellt. Zudem kann sie eine Körperhaltung finden, in welcher die Brusthaube dichtend und bequem an der Brust anliegt und der Milchsammelbehälter sie nicht stört. Insbesondere kann er beabstandet von ihrem Körper gehalten werden.

In einer Ausführungsform verläuft die erste Längsmittelachse in einem Winkel ungleich Null zur zweiten Längsmittelachse. Das heisst, dass keine Drehposition vorhanden ist, in welcher die erste und die zweite Längsmittelachse parallel zueinander verlaufen oder gar zusammenfallen. Vorzugsweise verlaufen die zwei Längsachsen in einer oder einigen wenigen Schwenkpositionen der Brusthaube parallel zueinander oder sie fallen sogar zusammen.

In bevorzugten Ausführungsformen existiert eine Position, in welcher die Brusthaube mit ihrer erweiterten Öffnung nach oben gedreht ist und eine Verlängerung des Milchsammelbehälters bildet. In dieser Position ist die Brusthaubeneinheit besonders standfest. In dieser Position lässt sich die Brusthaubeneinheit beispielsweise sicher auf einen Tisch stellen. In dieser Position verlaufen die zwei Längsmittelachsen vorzugsweise im kleinsten Winkel zueinander oder sie verlaufen parallel zueinander oder sie fallen zusammen.

In bevorzugten Ausführungsformen ist die erste Längsmittelachse auf einem imaginären ersten Kegelmantel um die Schwenkachse schwenkbar und/oder die zweite Längsmittelachse ist auf einem imaginären zweiten Kegelmantel um die Schwenkachse schwenkbar. Die erste oder zwei Längsmittelachse schneidet die Schwenkachse vorzugsweise in einem von 90° abweichenden Winkel, wenn sie entlang eines Kegelmantels um sie schwenkbar ist.

In einer anderen bevorzugten Ausführungsform ist die erste Längsmittelachse in einer zur Schwenkachse senkrecht verlaufenden Ebene um die Schwenkachse schwenkbar und/oder die zweite Längsmittelachse ist in einer zur Schwenkachse senkrecht verlaufenden Ebene um die Schwenkachse schwenkbar. Die erste oder zweite Längsmittelachse schneidet die Schwenkachse vorzugsweise in einem rechten Winkel, wenn sie in einer Ebene um sie schwenkbar ist.

Diese Ausführungsformen ermöglichen es der Brusthaube, die unterschiedlichsten Raumstellungen in Bezug auf einen annähernd vertikal ausgerichteten Milchsammelbehälter einzunehmen.

Damit die Brusthaubeneinheit gleichermassen zum Abpumpen der rechten wie der linken Brust eingesetzt werden kann, ist der erste Konnektorteil vorzugsweise in zwei einander entgegen gesetzte Richtungen relativ zum zweiten Konnektorteil schwenkbar.

Vorzugsweise ist das erste Konnektorteil um einen Winkel von 120° bis 260°, vorzugsweise von annähernd 240°, relativ zum zweiten Konnektorteil schwenkbar.

Um eine Montage und Reinigung zu erleichtern, ist das erste Konnektorteil vorzugsweise lösbar und wieder montierbar mit dem zweiten Konnektorteil verbunden.

Damit sich während des Abpumpens die einmal eingestellte Position der Brusthaube relativ zum Milchsammelbehälter nicht wieder verändert, sind das erste Konnektorteil und das zweite Konnektorteil vorzugsweise selbsthemmend relativ zueinander schwenkbar, so dass die Brusthaube und der Milchsammelbehälter in fixierten Positionen haltbar sind.

In bevorzugten Ausführungsformen erstreckt sich der Milchkanal in einem ersten Bereich entlang der ersten Längsmittelachse und in einem zweiten Bereich entlang der zweiten Längsmittelachse, wobei er in einem zwischen dem ersten und zweiten Bereich liegenden Zwischenbereich in einem Winkel zur ersten Längsmittelachse und zur zweiten Längsmittelachse verläuft. Der Verlauf des Milchkanals ändert sich dabei je nach Schwenkposition der Brusthaube, wobei er sich je nach Ausführungsform um die Schwenkachse bewegt. Vorzugsweise verläuft der Zwischenbereich entlang der Schwenkachse.

Vorzugsweise ist ein Sauganschluss zur Verbindung mit der Vakuumpumpe vorhanden, welcher im ersten Konnektorteil angeordnet ist. Der Sauganschluss wird somit von der Bewegung der Brusthaube nicht tangiert und verbleibt wie der Milchsammelbehälter in unveränderter Position relativ zum ersten Konnektorteil.

In bevorzugten Ausführungsformen ist im ersten Konnektorteil eine Kammer vorhanden, mit einer darin angeordneten Medientrennmembran zur Übertragung eines von der Vakuumpumpe zyklisch erzeugten Unterdrucks an die Brusthaube und zum Schutz der Vakuumpumpe vor Verunreinigung durch abgepumpte Milch. Diese Medientrennmembran trennt die Kammer in einen brusthaubenseitigen Bereich und einen vakuumseitigen Bereich. Sie ist üblicherweise nicht selber mechanisch angetrieben, sondern überträgt lediglich die auf sie einwirkenden Druckänderungen.

In einer bevorzugten Ausführungsform weist das erste Konnektorteil ein Basisteil und ein lösbar mit ihm verbundenes vakuumseitiges Teil auf, wobei im vakuumseitigen Teil der Sauganschluss angeordnet ist und wobei das Basisteil und das vakuumseitige Teil gemeinsam mit je einer einander gegenüberliegenden Wand die Kammer bilden. Die Wand des Basisteils ist im Wesentlichen geschlossen ausgebildet und weist lediglich eine zur Brusthaube führende Saugöffnung und in die Kammer gelangende Milch abführende Milchabflussöffnung auf, wobei die Milchabflussöffnung in den Milchkanal mündet.

In einer bevorzugten Ausführungsform ist die Medientrennmembran in einer Ebene angeordnet, welche in einem Winkel von 0° bis 20°, vorzugsweise annähernd parallel zur ersten Längsmittelachse und zur zweiten Längsmittelachse verläuft. Vorzugsweise verläuft sie annähernd parallel zur ersten Längsmittelachse und in einem spitzen Winkel von maximal 20° zur zweiten Längsmittelachse.

Der Konnektor einer Brusthaubeneinheit weist einen Sauganschluss zur Verbindung mit der Vakuumpumpe, einen Brusthaubenanschluss zur Verbindung mit der Brusthaube und einen Behälteranschluss zur Verbindung mit dem Milchsammelbehälter auf. Der Konnektor hat ferner einen Milchkanal, welcher vom Brusthaubenanschluss zum Behälteranschluss führt. Der Brusthaubenanschluss verläuft entlang einer ersten Längsmittelachse und der Behälteranschluss entlang einer zweiten Längsmittelachse. Der Brusthaubenanschluss ist relativ zum Behälteranschluss bewegbar. Er ist aus mindestens einem ersten Konnektorteil und einem zweiten Konnektorteil zusammengesetzt, wobei das erste Konnektorteil den Behälteranschluss aufweist und das zweite Konnektorteil den Brusthaubenanschluss aufweist. Das erste Konnektorteil ist um eine Schwenkachse relativ zum zweiten Konnektorteil schwenkbar. Diese Schwenkachse ist dabei weder die erste noch die zweite Längsmittelachse.

Vorzugsweise ist das zweite Konnektorteil in eine Schwenkposition relativ zum ersten Konnektorteil bringbar, in welcher die entlang des Brusthaubenanschlusses verlaufende zweite Längsmittelachse annähernd parallel zu der entlang des Behälteranschlusses verlaufenden ersten Längsmittelachse verläuft. Dieser Konnektor lässt sich mit unterschiedlich geformten Brusthauben und Milchsammelbehältern verwenden. Vorzugsweise ist der Milchsammelbehälter steif ausgebildet. Vorzugsweise ist er eine Säuglingsflasche aus Kunststoff oder Glas. Vorzugsweise ist dieser Konnektor im Wesentlichen kugelförmig ausgebildet. Vorzugsweise ist er an seinem Umfang mit einer oder mehreren Markierungen versehen, welche die Drehpositionen visualisieren und beispielsweise die Einstellung für ein linksseitiges und ein rechtsseitiges Anlegen an die Brust veranschaulichen.

Weitere Ausführungsformen sind in den abhängigen Ansprüchen angegeben.

### KURZE BESCHREIBUNG DER ZEICHNUNGEN

Eine bevorzugte Ausführungsform der Erfindung ist im Folgenden anhand der Zeichnungen beschrieben, die lediglich zur Erläuterung dienen und nicht einschränkend auszulegen sind. In den Zeichnungen zeigen:
- Figur 1: einen Längsschnitt durch eine erfindungsgemässe Brusthaubeneinheit mit eingezeichneten Achsen;
- Figur 2: die erfindungsgemässe Brusthaubeneinheit gemäss Figur 1 in verschiedenen Schwenkpositionen der Brusthaube;
- Figur 3: eine Mutter beim Abpumpen von Muttermilch in aufrecht sitzender Körperhaltung;
- Figur 4: die Mutter gemäss Figur 3 in zurückgelehnter Haltung;
- Figur 5: eine weitere Mutter beim Abpumpen von Milch in zurückgelehnter Haltung;
- Figur 6: eine Ansicht von zwei erfindungsgemässen Brusthaubeneinheiten gemäss Figur 1 bei Auflage auf einer linken und einer rechten Mutterbrust in einer ersten Schwenkposition;
- Figur 7: die zwei Brusthaubeneinheiten gemäss Figur 6 in einer zweiten Schwenkposition;
- Figur 8: eine perspektivische Darstellung der Brusthaubeneinheit gemäss Figur 1 in einer Position zum Abpumpen der rechten Mutterbrust;
- Figur 9: eine perspektivische Darstellung der Brusthaubeneinheit gemäss Figur 1 in einer Position zum Abpumpen der linken Mutterbrust;
- Figur 10: eine perspektivische Darstellung der Brusthaubeneinheit gemäss Figur 1 in einer Ruheposition;
- Figur 11a: eine Explosionsdarstellung eines erfindungsgemässen Konnektors der Brusthaubeneinheit gemäss Figur 1;
- Figur 11b: der Konnektor gemäss Figur 11a in einer anderen Darstellungsart;
- Figur 11c: einen Teilschnitt durch den Konnektor gemäss Figur 11b in einer anderen Perspektive;
- Figur 12: eine perspektivische Darstellung eines brusthaubenseitigen Teils des Konnektors gemäss Figur 11a;
- Figur 13a: eine Ansicht eines Basisteils des Konnektors gemäss Figur 11a von vorne;
- Figur 13b: das Basisteil gemäss Figur 13a in einer anderen Darstellungsart;
- Figur 14a: eine Ansicht des Basisteils des Konnektors gemäss Figur 11a von hinten;
- Figur 14b: das Basisteil gemäss Figur 14a in einer anderen Darstellungsart;
- Figur 15: eine Seitenansicht des Basisteils gemäss Figur 13a;
- Figur 16: einen Längsschnitt durch das zusammengesetzte Basisteil und das brusthaubenseitige Teil gemäss Figur 11a;
- Figur 17: einen Teilschnitt durch das Basisteil und das brusthaubenseitige Teil gemäss Figur 11 a in Explosionsdarstellung;
- Figur 18: den Teilschnitt gemäss Figur 17 mit zusammengesetztem Basisteil und brusthaubenseitigem Teil;
- Figur 19a: einige vergrösserte Darstellungen von Ausschnitten der Darstellung gemäss Figur 18 und
- Figur 19b: den Teilschnitt gemäss Figur 18 in einer anderen Darstellungsart.

### BESCHREIBUNG BEVORZUGTER AUSFÜHRUNGSFORMEN

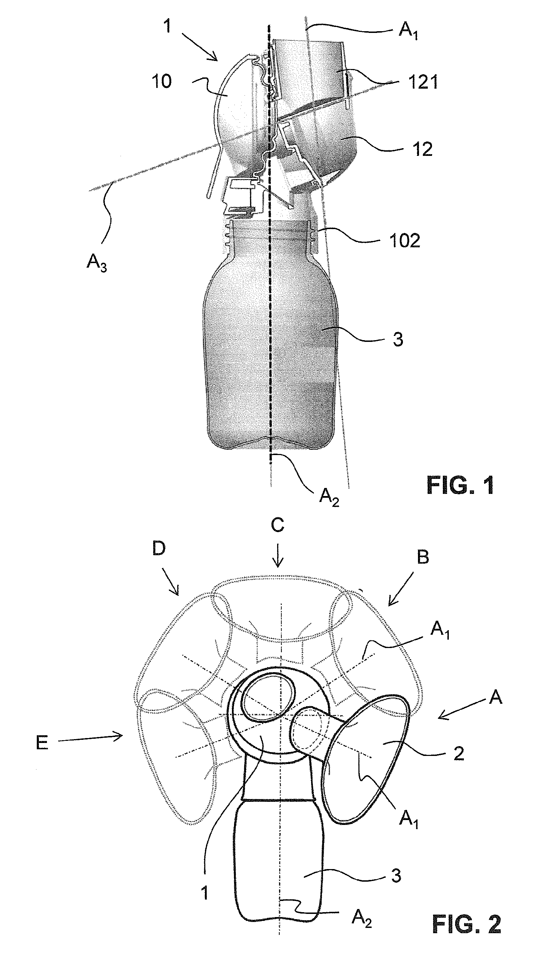

In den Figuren 1 bis 19b ist ein bevorzugtes Ausführungsbeispiel dargestellt. Dieses ist lediglich exemplarisch zu verstehen. Die erfindungsgemässen Prinzipien der zwei Längsmittelachsen und der Schwenkachse mit ihren Anordnungen zueinander, die in Bezug auf die Schwenkachse in einer Ebene oder über einen Kegelmantel sich bewegenden Längsmittelachsen sowie die annähernd parallele oder in einem kleinen spitzen Winkel zu den Längsmittelachsen angeordnete Medientrennmembran lassen sich auch auf andere Art und Weise verwirklichen.

In Figur 1 ist die erfindungsgemässe Brusthaubeneinheit ohne Brusthaube dargestellt. Figur 1 zeigt ein Kopplungselement, hier Adapter oder Konnektor 1 genannt, sowie einen Milchsammelbehälter 3. Der Konnektor 1 verbindet den Milchsammelbehälter 3 mit einer Brusthaube 2, wie sie in Figur 2 dargestellt ist.

Zudem dient der Konnektor 1 als Verbindungselement zu einer Vakuumpumpe. Entweder indem die Pumpe direkt am Konnektor befestigt bzw. in diesem integriert ist oder indem am Konnektor ein Saugschlauch anschliessbar ist, welcher zur Vakuumpumpe der Brustpumpe führt. Die Vakuumpumpe kann manuell oder mit einem Elektromotor betrieben werden.

Die Brusthaube kann eine beliebige Form aufweisen. Vorzugsweise entspricht ihre Form einer Brusthabe, wie sie in den am gleichen Tag von der Anmelderin eingereichten Patentanmeldungen mit den Titeln "Brusthaube mit flexiblem Rand" und "Brusthaube" dargestellt und beschrieben sind. Der Inhalt dieser zwei Patentanmeldungen wird hiermit durch Referenz in diesen Text aufgenommen.

Die Brusthaube 2 und eine in Figur 1 sichtbare Brusthaubenaufnahme 121 definieren eine erste Längsmittelachse A₁. Der Milchsammelbehälter 3 und Behälteranschluss 102 definieren eine zweite Längsmittelachse A₂. Eine Schwenkachse ist in Figur 1 mit dem Bezugszeichen A₃ versehen. Diese Schwenkachse A₃ verläuft durch den Konnektor 1, wobei zwei Teile 10, 12 des Konnektors 1 relativ zueinander schwenkbar sind. Dadurch lässt sich auch die Brusthaubenaufnahme 121 und die in ihr gehaltene Brusthaube 2 relativ zum Milchsammelbehälter 3 schwenken.

Die erste und die zweite Längsmittelachse A₁, A₂ können in einer Position parallel zueinander verlaufen oder vorzugsweise sogar zusammen fallen. In der hier dargestellten Position verlaufen sie beabstandet zueinander. Sie können zudem in allen Schwenkpositionen einen Winkel zueinander aufweisen, wobei dieser Winkel vorzugsweise relativ klein, vorzugsweise kleiner als 20° ist.

Die Schwenkachse A₃ schliesst mit den zwei Längsmittelachsen A₁, A₂ vorzugsweise einen rechten Winkel oder einen von 90° nicht allzu stark abweichenden Winkel ein, insbesondere von annähernd 70°. Die Winkel der zwei Längsmittelachsen A₁, A₂ zur Schwenkachse A₃ können unterschiedlich sein. Vorzugsweise sind sie jedoch betragsmässig gleich weit von 90° entfernt, jedoch mit verschiedenem Vorzeichen; z.B. beträgt der eine Winkel 70° und der andere 110°. Die zwei Längsmittelachsen A₁, A₂ schneiden die Schwenkachse A₃ vorzugsweise innerhalb des Konnektors 1.

Wie in Figur 1 erkennbar ist, bewegen sich die Längsmittelachsen A₁, A₂ während einer Schwenkbewegung auf einem imaginären Kegelmantel um die Schwenkachse A₃. Verläuft eine dieser Längsmittelachsen A₁, A₂ senkrecht zur Schwenkachse A₃, so bewegt sie sich in einer Ebene um die Schwenkachse A₃.

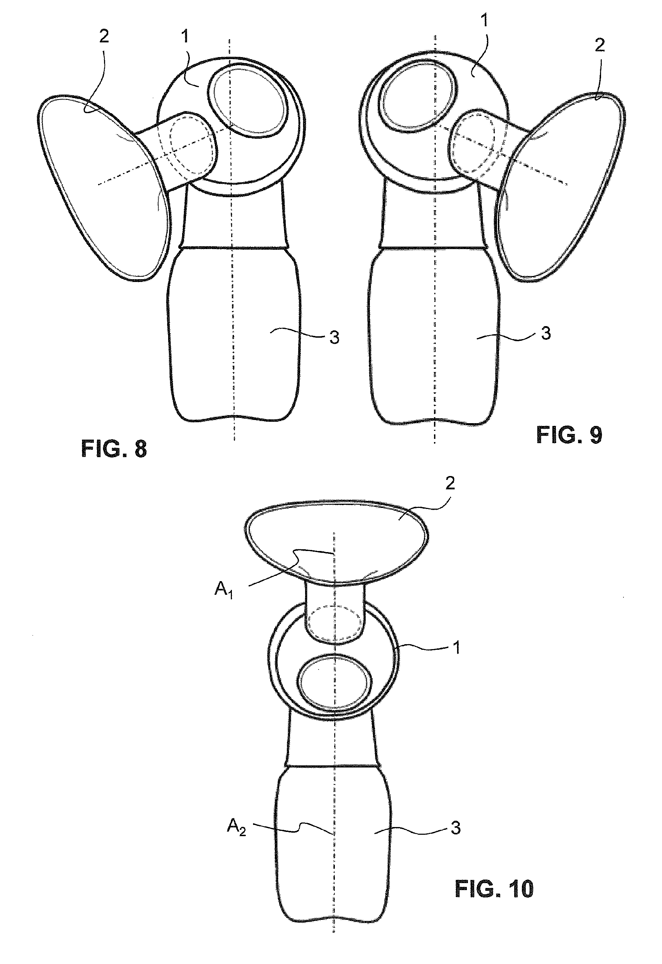

Wie in Figur 2 erkennbar ist, lässt sich die Brusthaube 2 dank der Schwenkachse A₃ in unterschiedliche Positionen in Bezug auf den Milchsammelbehälter 3 bringen. Die Position A stellt die Situation beim Abpumpen einer rechten Brust in zurückgelehnter Haltung der Mutter dar, die Position B beim Abpumpen der rechten Brust bei aufrechter Körperhaltung der Mutter. Die Position A ist auch in Figur 9 dargestellt. Wie in Figur 5 gut erkennbar ist, verläuft die zweite Längsmittelachse A₂ des Milchsammelbehälters 3 in dieser Position A auch bei zurückgelehnter Haltung der Mutter annähernd vertikal, d.h. der Milchsammelbehälter 3 ist annähernd aufrecht.

Die Positionen D und E gemäss Figur 2 entsprechen denselben Situationen beim Abpumpen der linken Brust. Die Position E ist auch in Figur 8 dargestellt.

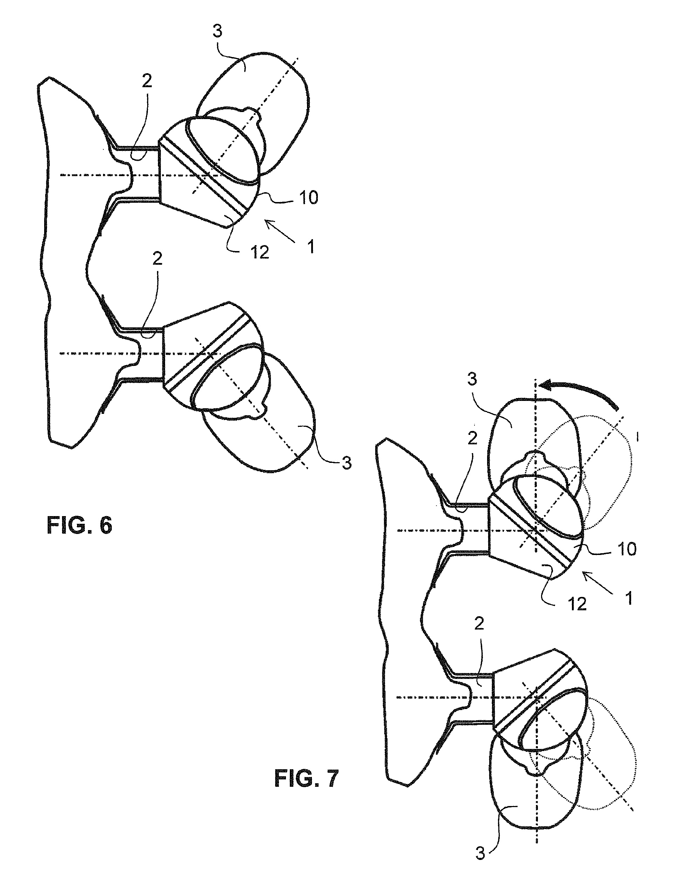

In den Figuren 6 und 7 ist erkennbar, wie sich die Lage des Milchsammelbehälters 3 in Bezug auf die Lage der Brusthaube 2 verändert, wenn die zwei Teile des Konnektors 1 zueinander verdreht werden. Dabei bleibt der Behälter 3 annähernd aufrecht.

In der Position C verlaufen die zwei Längsmittelachsen A₁, A₂ parallel zueinander. Noch bevorzugter fallen sie zusammen. Dies ist in Figur 10 dargestellt. In dieser Position C lässt sich die Brusthaube 2 auf einfache Art und Weise in den Konnektor 1 einstecken. Zudem ist dies die stabilste Position, wenn die Einheit auf einen Tisch gestellt ist. Die Vorrichtung fällt nicht um. Des Weiteren kann dadurch Milch, welche sich noch in der Brusthaube 2 befindet, in den Milchsammelbehälter 3 abfliessen.

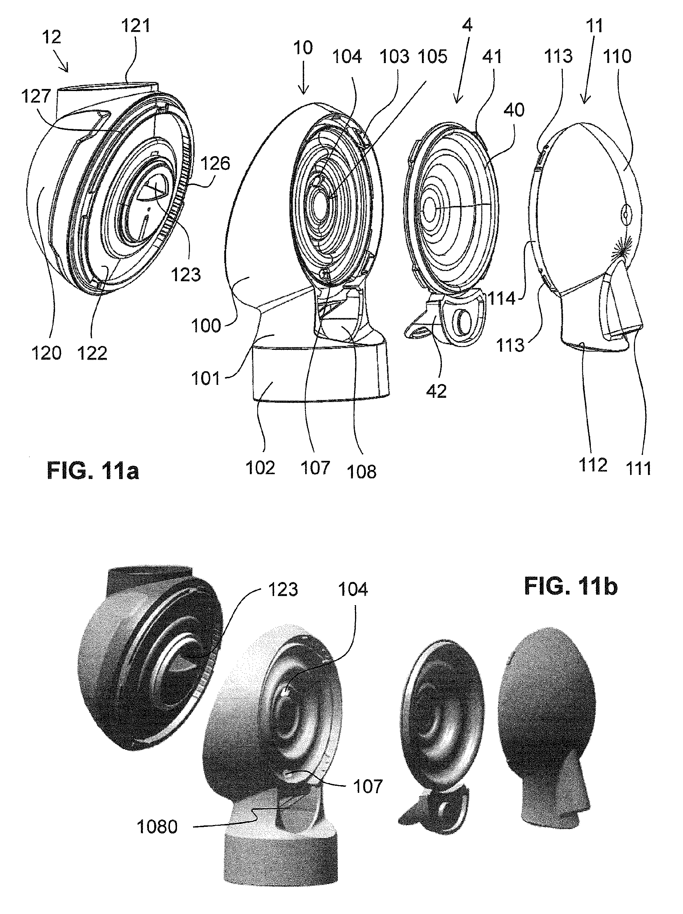

In den Figuren 11a bis 19b ist der Konnektor 1 des exemplarischen Ausführungsbeispiels detailliert dargestellt.

Der Konnektor 1 weist zwei Teile auf, welche gegeneinander verdrehbar sind: das genannte Basisteil 10 mit einem vakuumseitigen Teil 11 einerseits und das genannte brusthaubenseitige Teil 12 andererseits. Dies ist in den Figuren 11a bis 11c gut erkennbar.

Das Basisteil 10 weist einen Grundkörper 100 auf, an welchem auf einer Seite das brusthaubenseitige Teil 12 schwenkbar und auf der gegenüberliegenden Seite das vakuumseitige Teil 11 fest verbindbar ist. Der Grundkörper 100 weist eine Kammer 103 auf, welche vom vakuumseitigen Teil 11 verschlossen ist. In dieser Kammer 103 ist eine Medientrennmembran 4 angeordnet. Basisteil 10, vakuumseitiges Teil 11 und brusthaubenseitiges Teil 12 sind vorzugsweise aus einem steifen Kunststoff gefertigt. Die Medientrennmembran 4 ist flexibel und vorzugsweise aus Silikon gefertigt.

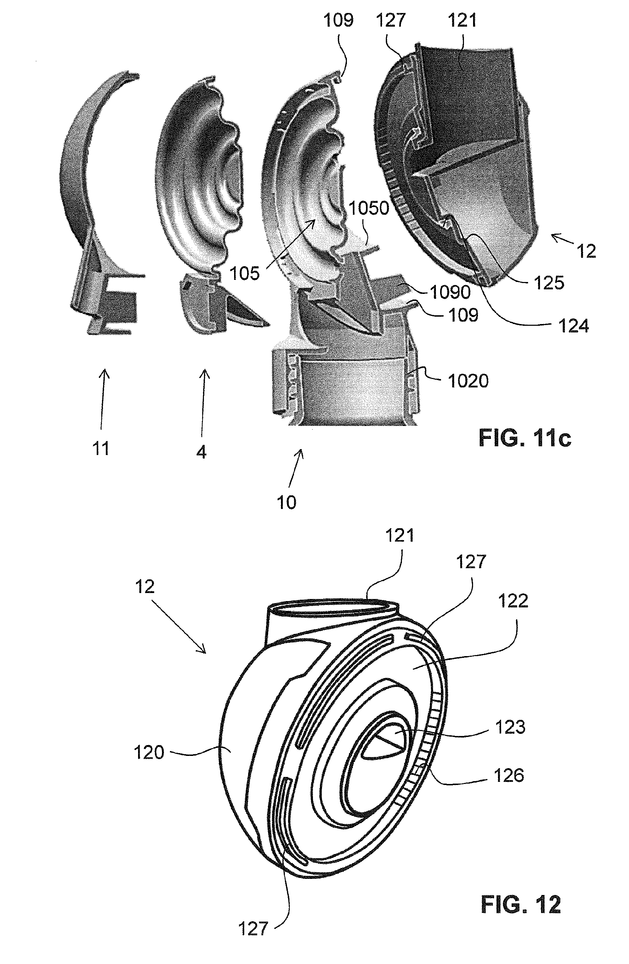

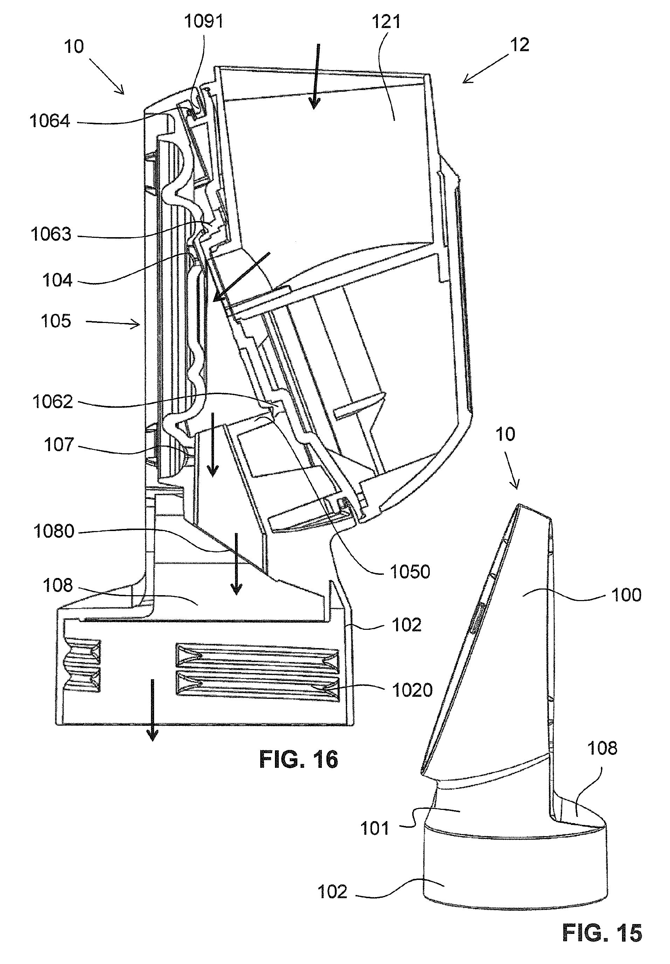

Der Grundkörper 100 geht an seiner unteren Seite in einen Hals 101 über, welcher in einem Behälteranschluss 102 endet, wie dies in Figur 15 dargestellt ist. Der Behälteranschluss 102 ist als rohrförmiger Stutzen ausgebildet mit einem Innengewinde 1020 (siehe Figur 11 c). Der Milchsammelbehälter 3 weist einen entsprechenden Hals mit einem Aussengewinde zur lösbaren Befestigung am Basisteil 10 auf. Andere Verbindungsarten sind ebenfalls möglich.

Das vakuumseitige Teil 11 weist ebenfalls einen Grundkörper 110 auf, an welchem einerseits ein Hals 112 angeformt ist, welcher das Gegenstück zum Hals 101 des Basisteils 10 bildet und diesen verschliesst. Ferner weist das vakuumseitige Teil 11 einen Sauganschluss 111 auf zur Verbindung mit einer Vakuumpumpe oder bevorzugter mit einem zur Vakuumpumpe führenden Saugschlauch. Ein dem Sauganschluss 111 entgegen gesetzten Ende des vakuumseitigen Teils 11 ist vorzugsweise rund ausgebildet und durch einen Kopplungsring 114 gebildet. Am äusseren Umfang des Kopplungsrings 114 sind vorzugsweise Einrastnasen 113 vorhanden.

Die Medientrennmembran 4 weist einen runden Grundkörper 40 auf, an dessen Umfang Positioniernasen 41 angeformt sind, welche in entsprechende Ausnehmungen 103 des Basisteils 10 eingreifen. Der Grundkörper 40 weist vorzugsweise umlaufende und in sich geschlossene Erhebungen und Vertiefungen auf, welche die Bewegung der Medientrennmembran 4 und somit die Übertragung eines über den Sauganschluss 111 in die Kammer 105 eingebrachten, sich zyklisch verändernden Unterdrucks an die Brusthaube 2 gewährleistet. Die Medientrennmembran 4 trennt die Kammer 105 in einen vakuumseitigen Bereich und einen brusthaubenseitigen Bereich, so dass die Pumpe vor der abgepumpten Milch und allfälligen Bakterien geschützt werden kann.

Eine Wand des Basisteils 10 bildet mit entsprechend ausgebildeten Vertiefungen und Erhebungen ein Gegenstück zur Medientrennmembran 4. In der Kammer 105 bzw. in dieser Wand ist eine Saugöffnung 104 vorhanden, welche die Kammer 105 mit einer Saugöffnung 123 des brusthaubenseitigen Teils 12 verbindet. Diese Saugöffnung 123 führt zum Brusthaubenanschluss 121.

Im unteren Bereich geht der Grundkörper 40 der Medientrennmembran 4 in eine Ventilklappe 42 über. Der zugehörige Ventilsitz 1080 eines Rückschlagventils ist durch den in den Hals 101 des Basisteils 10 ragenden Milchkanal 108 gebildet. Dieses Rückschlagventil verschliesst den Zugang zum Milchsammelbehälter und reduziert somit das Totvolumen.

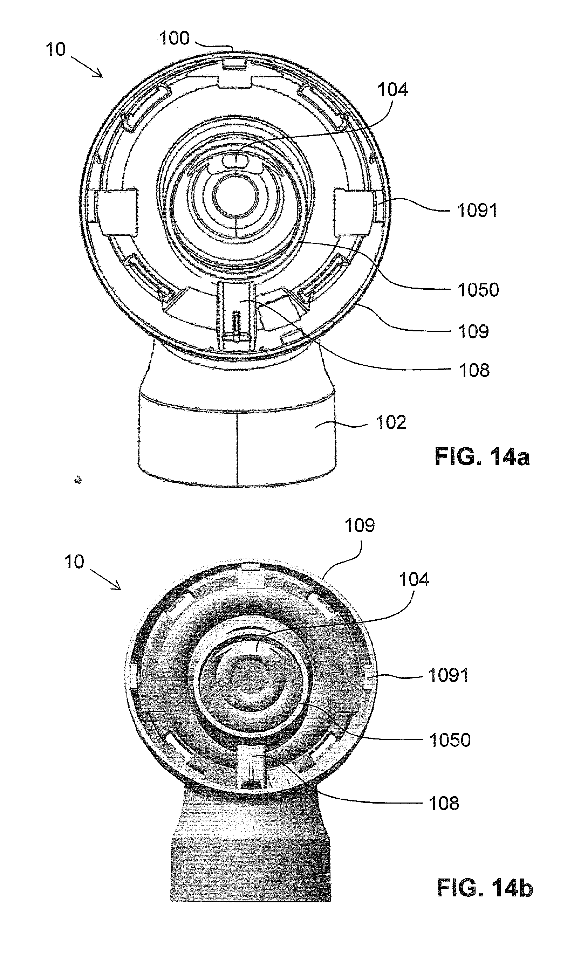

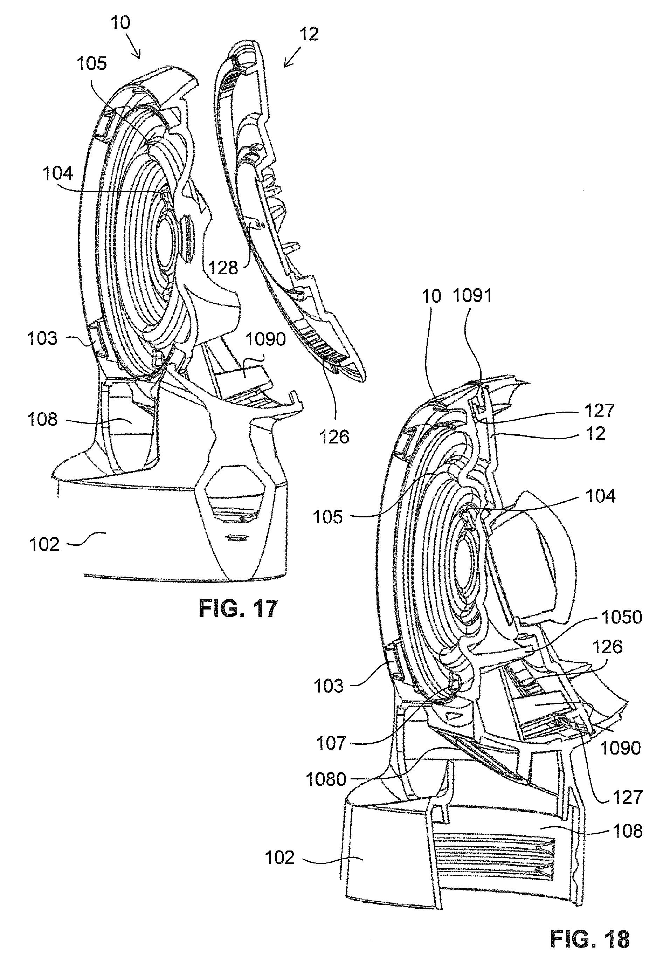

Das brusthaubenseitige Teil 12 weist, wie auch in Figur 12 erkennbar ist, ebenfalls einen Grundkörper 120 auf mit einer Deckfläche 122, welche vorzugsweise bis auf die Saugöffnung 123 verschlossen ist und welche dem Basisteil 10 zugewandt ist. Die Deckfläche 122 ist von einem Positionierring 127 umgeben, welcher teilweise durchbrochen ist. Dieser Positionierring 127 lässt sich in einem umlaufenden Rand 109 mit entsprechenden Einrastelementen 1091 des Basisteils 10 verbinden, wobei er schwenkbar darin gehalten ist (siehe Figuren 14a und 14b). Der Positionierring 127 definiert somit die Schwenkachse A₃, welche die Längsmittelachse dieses Rings 127 bildet. Innerhalb dieses Rings 127 sind Positionsnuten 126 vorhanden, in welche ein entsprechendes Hemmelement 1090 des Basisteils 10 eingreift, so dass selbsthemmende Schwenkbarkeit des ersten Konnektorteils 1 ermöglicht ist. Dies ist auch in den Figuren 17, 18 und 19b gut erkennbar.

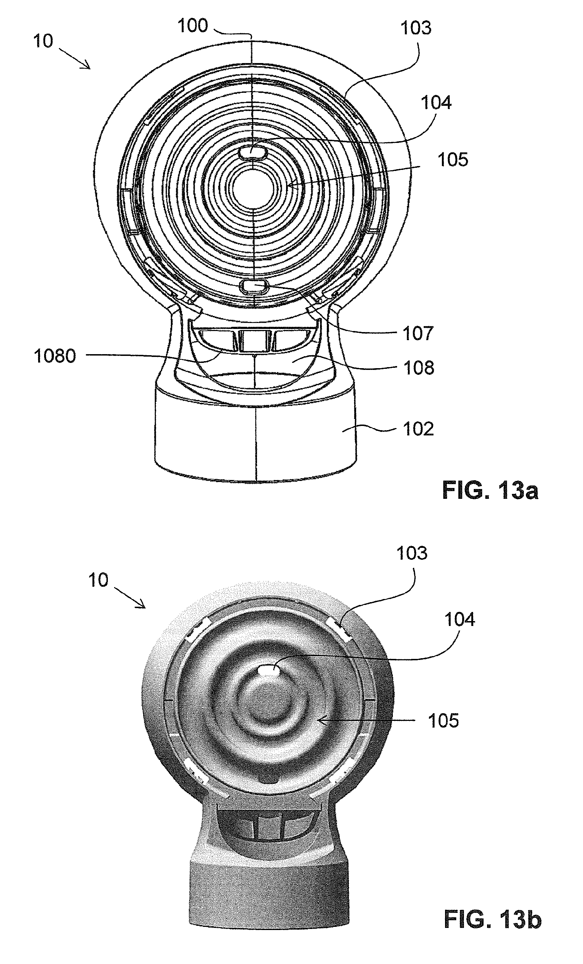

Die Saugöffnung 104 in der Kammer 105 ist relativ klein ausgebildet. Im unteren Bereich der zur Saugöffnung 104 gehörenden Wand der Kammer 105 ist eine Milchabflussöffnung 107 vorhanden. Dies ist in den Figuren 13a und 13b gut erkennbar. Sollte abgepumpte Milch durch die Saugöffnung 104 in die Kammer 105 gelangen, so kann sie durch diese Öffnung 107 und das Rückschlagventil in den Milchkanal 108 und somit in den Milchsammelbehälter 3 fliessen.

Der eigentliche Milchkanal 108 verläuft vom rohrförmigen Brusthaubenanschluss 121 über die Saugöffnung 123 des brusthaubenseitigen Teils 12 entlang der der Kammer 105 entgegenliegenden Seite der Kammerwand, also vor der Saugöffnung 104 des Basisteils 10 in den Hals 101 und dem Behälteranschluss 102 des Basisteils 10. Dies ist in den Figuren 14a und 14b gut erkennbar. In Figur 16 ist der Milchfluss im Milchkanal 108 mit Pfeilen dargestellt. Wie erkennbar ist, verläuft der Milchkanal 108 nicht geradlinig, sondern weist eine Umlenkung von 70° bis 150° auf.

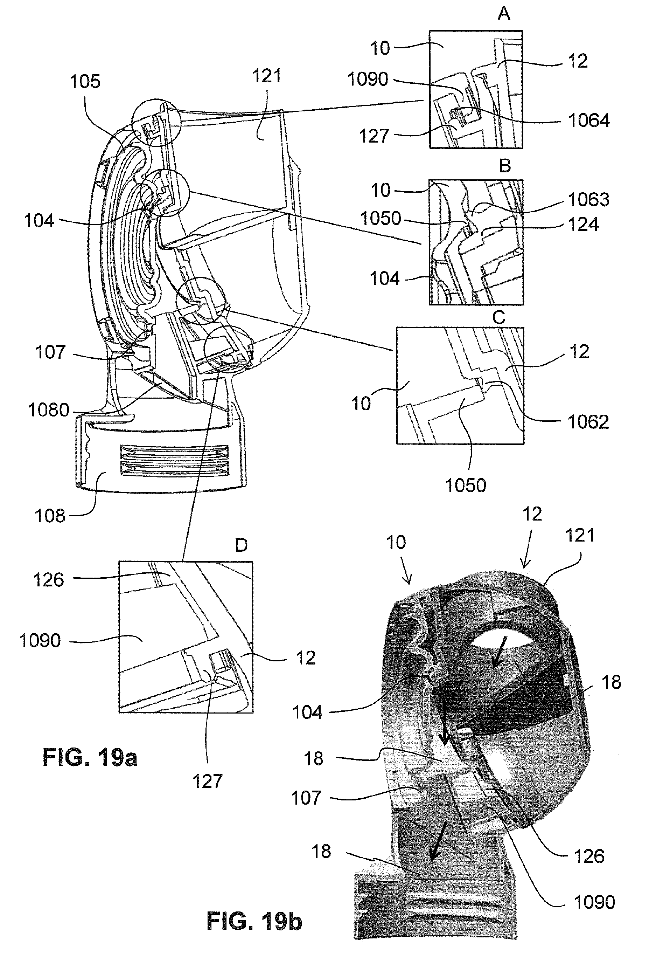

Der Konnektor 1 bildet einen Rotations- oder Schwenkkopf, welcher in jeder Schwenkposition dicht ist. In Figur 16 sind angespritzte Dichtungsringe mit den Bezugszeichen 1062, 1063 und 1064 versehen. Der azentrische Dichtungsring ist mit dem Bezugszeichen 1050 versehen und in den Figuren 14a und 14b sowie 19a gut erkennbar.

Der schwenkbare und als Rotationskopf ausgebildete Konnektor ermöglicht ein Abpumpen bei zurückgelehnter Körperhaltung der Mutter in diversen Positionen der Brusthaube, wobei der Milchsammelbehälter in einer annähernd aufrechten Position verbleibt.

### BEZUGSZEICHENLISTE

| | | | |
|---|---|---|---|
| 1 | Konnektor | 120 | Grundkörper |
| | | 121 | Brusthaubenanschluss |
| 10 | Basisteil | 122 | Deckfläche |
| 100 | Grundkörper | 123 | Saugöffnung |
| 101 | Hals | 126 | Positionsnuten |
| 102 | Behälteranschluss | 127 | Positionierring |
| 1020 | Innengewinde | | |
| 103 | Ausnehmung | 2 | Brusthaube |
| 104 | Saugöffnung | | |
| 105 | Kammer | 3 | Milchsammelbehälter |
| 1050 | azentrischer Dichtungsring | | |
| 1062 | Dichtungsring | 4 | Medientrennmembran |
| 1063 | Dichtungsring | 40 | Grundkörper |
| 1064 | Dichtungsring | 41 | Positioniernase |
| 107 | Milchabflussöffnung | 42 | Ventilklappe |
| 108 | Milchkanal | | |
| 1080 | Ventilsitz | A₁ | erste Schwenkachse |
| 109 | Rand | A₂ | zweite Schwenkachse |
| 1090 | Hemmelement | A₃ | dritte Schwenkachse |
| 1091 | Einrastelement | | |
| | | A | linke aufrechte |
| 11 | vakuumseitiges Teil | | Abpumpposition |
| 110 | Grundkörper | B | linke zurückgelehnte |
| 111 | Sauganschluss | | Abpumpposition |
| 112 | Hals | C | Ruheposition |
| 113 | Einrastnasen | D | rechte zurückgelehnte |
| 114 | Kopplungsring | | Abpumpposition |
| | | E | rechte aufrechte |
| 12 | brusthaubenseitiges Teil | | Abpumpposition |

## Patentansprüche

1. Brusthaubeneinheit zur Verwendung in einer Vorrichtung zum Abpumpen von menschlicher Muttermilch, wobei die Brusthaubeneinheit eine Brusthaube (2) zur Anlage an eine Mutterbrust, einen Milchsammelbehälter (3) zur Aufnahme der abgepumpten Muttermilch und einen Konnektor (1) zur Verbindung der Brusthaube (2) mit einer Vakuumpumpe der Vorrichtung und mit dem Milchsammelbehälter (3) aufweist, wobei der Konnektor (1) einen Milchkanal (108) aufweist, welcher von der Brusthaube (2) zum Milchsammelbehälter (3) führt, wobei die Brusthaube (2) eine erste Längsmittelachse (A₁) aufweist und der Milchsammelbehälter (3) eine zweite Längsmittelachse (A₂) aufweist, und wobei die mit dem Konnektor (1) verbundene Brusthaube (2) relativ zum ebenfalls mit dem Konnektor (1) verbundenen Milchsammelbehälter (3) bewegbar ist,
**dadurch gekennzeichnet,**
**dass** der Konnektor (1) aus mindestens einem ersten Konnektorteil (10, 11) und einem zweiten Konnektorteil (12) zusammengesetzt ist,
**dass** das erste Konnektorteil (10, 11) den Milchsammelbehälter (3) hält und das zweite Konnektorteil (12) die Brusthaube (2) hält und
**dass** das erste Konnektorteil (10, 11) um eine Schwenkachse (A₃) relativ zum zweiten Konnektorteil (12) schwenkbar ist, wobei diese Schwenkachse (A₃) weder die erste noch die zweite Längsmittelachse (A₁, A₂) ist.

2. Brusthaubeneinheit nach Anspruch 1, wobei die erste Längsmittelachse (A₁) in einem Winkel ungleich Null zur zweiten Längsmittelachse (A₂) verläuft.

3. Brusthaubeneinheit nach einem der Ansprüche 1 oder 2, wobei die erste Längsmittelachse (A₁) auf einem imaginären ersten Kegelmantel um die Schwenkachse (A₃) schwenkbar ist und/oder wobei die zweite Längsmittelachse (A₂) auf einem imaginären zweiten Kegelmantel um die Schwenkachse (A₃) schwenkbar ist.

4. Brusthaubeneinheit nach einem der Ansprüche 1 oder 2, wobei die erste Längsmittelachse (A₁) in einer zur Schwenkachse (A₃) senkrecht verlaufenden Ebene um die Schwenkachse (A₃) schwenkbar ist und/oder wobei die zweite Längsmittelachse (A₂) in einer zur Schwenkachse (A₃) senkrecht verlaufenden Ebene um die Schwenkachse (A₃) schwenkbar ist.

5. Brusthaubeneinheit nach einem der Ansprüche 1 bis 4, wobei das erste Konnektorteil (10, 11) in zwei einander entgegen gesetzte Richtungen relativ zum zweiten Konnektorteil (12) schwenkbar ist.

6. Brusthaubeneinheit nach einem der Ansprüche 1 bis 5, wobei das erste Konnektorteil (10, 11) um einen Winkel von 120° bis 260°, vorzugsweise von annähernd 240°, relativ zum zweiten Konnektorteil (12) schwenkbar ist.

7. Brusthaubeneinheit nach einem der Ansprüche 1 bis 6, wobei das erste Konnektorteil (10, 11) lösbar und wieder montierbar mit dem zweiten Konnektorteil (12) verbunden ist.

8. Brusthaubeneinheit nach einem der Ansprüche 1 bis 7, wobei das erste Konnektorteil (10, 11) und das zweite Konnektorteil (12) selbsthemmend relativ zueinander schwenkbar sind, so dass die Brusthaube (2) und der Milchsammelbehälter (3) in fixierten Positionen haltbar sind.

9. Brusthaubeneinheit nach einem der Ansprüche 1 bis 8, wobei sich der Milchkanal (18) in einem ersten Bereich entlang der ersten Längsmittelachse (A₁) und in einem zweiten Bereich entlang der zweiten Längsmittelachse (A₂) erstreckt und wobei er in einem zwischen dem ersten und zweiten Bereich liegenden Zwischenbereich in einem Winkel zur ersten Längsmittelachse (A₁) und zur zweiten Längsmittelachse (A₂) verläuft.

10. Brusthaubeneinheit nach Anspruch 9, wobei der Zwischenbereich entlang der Schwenkachse (A₃) verläuft.

11. Brusthaubeneinheit nach einem der Ansprüche 1 bis 10, wobei ein Sauganschluss (111) zur Verbindung mit der Vakuumpumpe vorhanden ist, welcher im ersten Konnektorteil (11) angeordnet ist.

12. Brusthaubeneinheit nach einem der Ansprüche 1 bis 11, wobei im ersten Konnektorteil (10, 11) eine Kammer (105) vorhanden ist mit einer darin angeordneten Medientrennmembran (4) zur Übertragung eines von der Vakuumpumpe zyklisch erzeugten Unterdrucks an die Brusthaube (2) und zum Schutz der Vakuumpumpe vor Verunreinigung durch abgepumpte Milch.

13. Brusthaubeneinheit nach den Ansprüche 11 und 12, wobei das erste Konnektorteil ein Basisteil (10) und ein lösbar mit ihm verbundenes vakuumseitiges Teil (11) aufweist, wobei im vakuumseitigen Teil (11) der Sauganschluss (111) angeordnet ist und wobei das Basisteil (10) und das vakuumseitige Teil (11) gemeinsam mit je einer einander gegenüberliegenden Wand die Kammer (105) bilden und wobei die Wand des Basisteils (10) im Wesentlichen geschlossen ausgebildet ist und lediglich eine zur Brusthaube (2) führende Saugöffnung (104) und in die Kammer (105) gelangende Milch abführende Milchabflussöffnung (107) aufweist, wobei die Milchabflussöffnung in den Milchkanal (108) mündet.

14. Brusthaubeneinheit nach einem der Ansprüche 12 oder 13, wobei die Medientrennmembran (4) in einer Ebene angeordnet ist, welche in einem Winkel von 0° bis 20°, vorzugsweise annähernd parallel, zur ersten Längsmittelachse (A₁) und zur zweiten Längsmittelachse (A₂) verläuft.

15. Konnektor einer Brusthaubeneinheit gemäss einem der Ansprüche 1 bis 13, wobei der Konnektor (1) einen Sauganschluss (111) zur Verbindung mit der Vakuumpumpe, einen Brusthaubenanschluss (121) zur Verbindung mit der Brusthaube (2) und einen Behälteranschluss (102) zur Verbindung mit dem Milchsammelbehälter (3) aufweist, wobei der Konnektor (1) einen Milchkanal (108) aufweist, welcher vom Brusthaubenanschluss (121) zum Behälteranschluss (102) führt, wobei der Brusthaubenanschluss (121) entlang der ersten Längsmittelachse (A₁) verläuft und der Behälteranschluss (102) entlang der zweiten Längsmittelachse (A₂) verläuft, und wobei der Brusthaubenanschluss (121) relativ zum Behälteranschluss (102) bewegbar ist,
**dadurch gekennzeichnet,**
**dass** der Konnektor (1) aus mindestens einem ersten Konnektorteil (10, 11) und einem zweiten Konnektorteil (12) zusammengesetzt ist,
**dass** das erste Konnektorteil (10, 11) den Behälteranschluss (102) aufweist und das zweite Konnektorteil (12) den Brusthaubenanschluss (121) aufweist und
**dass** das erste Konnektorteil (10, 11) um eine Schwenkachse (A₃) relativ zum zweiten Konnektorteil (12) schwenkbar ist, wobei diese Schwenkachse (A₃) weder die erste noch die zweite Längsmittelachse (A₁, A₂) ist.
